# EUROPEAN PATENT SPECIFICATION

(11) **EP 4 419 539 B1**
(45) Date of publication and mention of the grant of the patent: **09.12.2026**
(21) Application number: 22884148.2
(22) Date of filing: 18.10.2022
(51) Int. Cl.: C07K 7/06, A61P 25/04, A61P 29/00, A61K 38/00

(54) **PEPTIDE AND ITS USES**
PEPTID UND DESSEN VERWENDUNGEN
PEPTIDE ET SES UTILISATIONS

(30) Priority: 18.10.2021 PL 43924321
(43) Date of publication of application: 28.08.2024
(73) Proprietor: Instytut Medycyny Doswiadczalnej I Klinicznej Im. Miroslawa Mossakowskiego Polskiej Akademii Nauk, 02-106 Warszawa (PL); Uniwersytet Warszawski, 00-927 Warszawa (PL)
(72) Inventor: KOSSON, Piotr, 0-892 Warszawa (PL); MATALINSKA, Joanna Aleksandra, 04-185 Warszawa (PL); DYNIEWICZ, Jolanta, 05-509 Józefoslaw (PL); MISICKA-KESIK, Aleksandra, 05-820 Piastów (PL); TYMECKA, Dagmara, 02-117 Warszawa (PL)
(74) Representative: Witek, Rafal
(86) International application number: PCT/PL2022/050068
(87) International publication number: WO 2023/068949

(56) References cited:
- EP-A2- 0 350 221
- WO-A2-2006/064530
- PL-B1- 202 928

## Description

The invention relates to an analgesic peptide which is particularly suitable for long-term use in the treatment of chronic pain.

Treatment of pain, especially severe chronic pain, is still an unsolved, and at the same time neglected problem of modern medicine. The pain management algorithm is based on the principle that the more severe the pain, the stronger pain medication it requires. This strategy is commonly known as the analgesic ladder, with non-opioid drugs on the first step, followed on the consecutive steps by weak opioids and finally opioid analgesics. This strategy includes also the use of adjunctive drugs (adjuvants) that may enhance the effects of opioids (so-called coanalgesics). Due to the lack of other equally effective analgesics, opioids are increasingly used in therapy. Currently, they are used not only to combat cancer pain or postoperative pain, but also to alleviate other pain conditions, e.g. in rheumatoid arthritis.

Pain signal resulting from a damage of body organs, or a disease is transmitted to the central nervous system, where it generates a pain sensation. The magnitude of the pain stimulus is regulated by the system of nociceptive and antinociceptive receptors located on the membrane of nerve cells. Endogenous opioid peptides are one of the natural factors that reduce the pain signal as a result of activation of antinociceptive receptors. These receptors are also activated by administration of opioid analgesics such as morphine or fentanyl. Unfortunately, administration of non-peptide analgesics causes a number of adverse reactions, including drug tolerance and drug addiction.

Limitations in the analgesic efficacy of opioids, related primarily to development of tolerance, resistance of neuropathic pain to the analgesic effect of opioids, or the so-called "paradoxical pains" after the use of opioids, form a barrier that could not be crossed yet. Another problem limiting the use of opioids is related to their side effects. These include, among others: persistent constipation, nausea, central symptoms such as consciousness disorders, cognitive disorders or respiratory depression (especially in case of overdosing). Especially, the development of tolerance to administered opioids is a serious limitation in their use in the treatment of severe chronic pain.

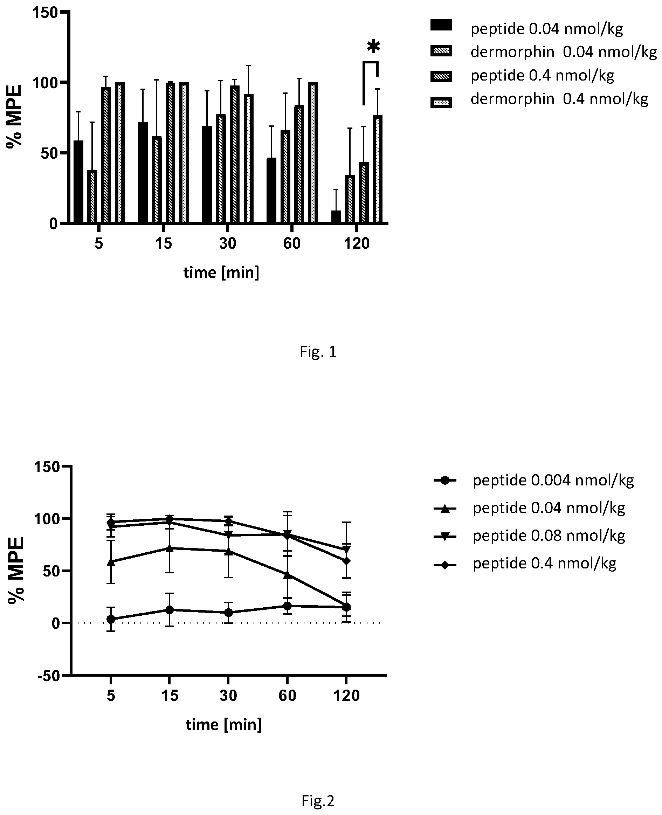

An alternative solution is provided by emploing peptide analogs of natural opioid peptides. The use of a peptide analog of natural opioid peptides, e.g. biphalin, permits to reduce formation of drug tolerance and drug addiction. Unfortunately, the conducted research has shown that opioid peptides have a limited ability to cross the blood-central nervous system barrier. According to data from experiments carried out on animals and presented in 1989 at the International Narcotic Research Conference 1989, and described in the conference materials, by B.S. Silbert, A.W. Lipkowski, D.B. Carr, S.K. Szyfelbein, P.F. Osgood, in the chapter "Peptides as potential nociceptive drugs.", pages 485-488 of the book Procc. Int. Narc. Res. Conf.'89 ", edited by R. Quirion, published by Alan R. Liss Inc, New York, in 1990, the effect of the opioid peptide with the chemical formula shown in Figure 2 (commonly named biphaline) after intravenous administration corresponds to doses of morphine. However, as described by D. Kosson, I. Maszczynska Bonney, D.B. Carr, E. Mayzner-Zawadzka, A.W. Lipkowski, in the article Antinociceptive properties of biphalin after intrathecal application in rats: a reevaluation. in Pharmacological Report, volume 57, pages 545-549, in 2005, bypassing the blood-central nervous system barrier by administration directly into the subarachnoid space causes that biphalin is more than one thousand times more active than morphine being administered by the same method. This means that in healthy individuals, the blood-central nervous system barrier is very effective in preventing biphalin from entering the central nervous system. The high activity of biphalin administered directly to the central nervous system was the basis for the use of biphalin as a drug for administration using modern methods of topical administration, which was described in the Polish patent application filed in 2002, application number P355470, by A.W. Lipkowski, D.B. Carr, I. Bonney, D. Kosson, A. Misicka-Kesik, and untitled " *Zastosowanie peptydów o dzia* 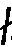 *aniu analgetycznym do podania bezpośrednio do miejsca ich spodziewanego dzia* 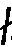 *ania przeciwbólowego (Use of analgesic peptides to be administered directly to the site of their expected analgesic activity)".*

Chronic pain is usually associated with pathological inflammation caused by a variety of factors, such as rheumatism, gout, post-accident or post-operative neurodegeneration, or cancer. Such changes in the organism result in, inter alia, a change in the permeability of biological barriers, including barriers separating the blood and the central nervous system, generally referred to as the "blood-brain barrier". The changes are discussed in the review by W. Pan, entitled "Permeability of the blood-brain barrier to neurotropic peptides", pages 1435-1442, in the monograph "Handbook of Biologically Active Peptides", edited by A.J. Kastin, published by Academic Press, Burlingston, in 2006.

European patent EP2195006B1 discloses the use of biphaline and its analogs as peripherally administered analgesic drug in the treatment of patients with a blood-brain barrier damage, occurring during a disease state associated with an inflammatory condition. Said drug is used at a subliminal dose, said subliminal dose is effective in sick patients, and has no analgesic effect in healthy subjects, and is lower than 1 mg/kg.

Dermorphin is a known neuropeptide of the formula shown below, and was isolated first from the skin of frogs belonging to the genus *Phyllomedusa.*

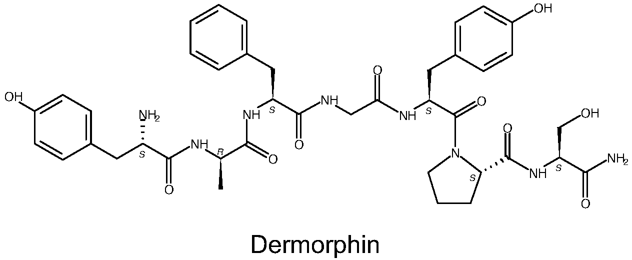

This peptide is an agonist at the mu opioid receptor (MOR) and is approximately 750-2200 times more potent than morphine ((M. Br. Br. Br. J. Pharmac. (1981) 73, 625-631), depending on the route of administration and the choice of an assay measuring the level of analgesia. However, applicability of the peptide in pharmacy is significantly limited by its low stability in the body.

The aim of the invention is to provide an analgesic drug that could be used for long-term analgesic therapy, especially in the treatment of severe chronic pain, and at the same time is deprived of the drawbacks associated with the use of opioids, such as the described above side effects occurring after the use of opioids, especially development of tolerance during the chronic administration.

Another aim is to provide a drug that could be used at a much lower dose than opioids such as morphine.

Still another aim of the invention is to provide an analgesic drug characterized by stability in the patient's body much higher than that of the known neuropeptides.

Unexpectedly, the complex technical problem defined in this way has been solved in the present invention.

The invention relates to a peptide and its uses as defined in the appended claims.

Unexpectedly, the peptide according to the invention is suitable for long-term use in the treatment of chronic pain without induction of drug tolerance, and at a dose at least 100 times lower than the analgesic effective dose of morphine in this patient.

Equally surprisingly, the peptide according to the invention, having analgesic activity similar to that of dermorphin, is much more stable than dermorphin itself, which constitute its significant advantage in comparison to the neuropeptide known from the state of the art.

For a better understanding of the invention, it has been illustrated with the following examples and figures.

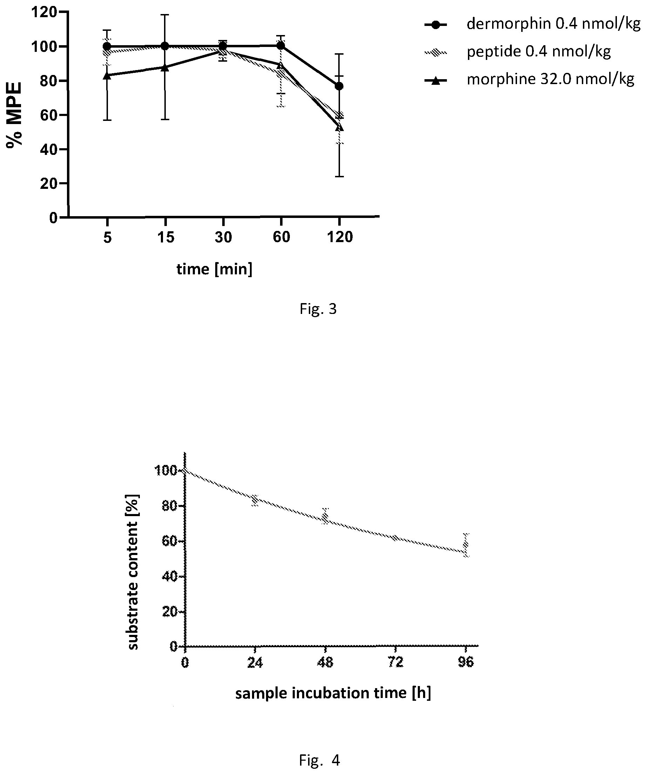

Figures 1, 2, and 3 show analgesic effect of the peptide according to the invention as compared to dermorphin and morphine. Figure 1 shows analgesic effect of the peptide after central i.t. application in an acute pain model in rats, Figure 2 shows analgesic effect of the peptide after central i.t. application in an acute pain model in rats, while Figure 3 shows analgesic effect of the peptide after central i.t. application compared to equipotent doses of dermorphin and morphine in an acute pain model in rats.

Figure 4 shows metabolic stability of the peptide in human serum.

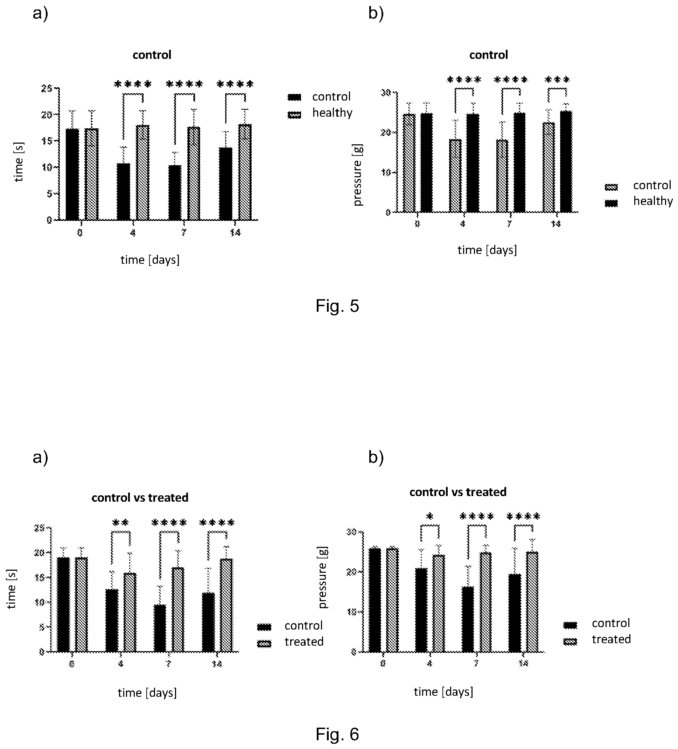

Figure 5 shows the effect of sciatic nerve ligation on the level of mechanical hypersensitivity, tested with the von Frey test in control rats versus a group of healthy rats without sciatic nerve ligation, a) duration of the irritant action expressed in seconds [s], b) pressure of the irritant stimulus expressed in grams [g]. Significance level p***<0.0003 p****<0.0001.

Figure 6 shows analgesic effect of the compound following central i.t. administration, in a chronic pain model involving ligation of the sciatic nerve, and measurement of the level of mechanical hypersensitivity tested with the von Frey test in rats in the control group, in relation to the group of rats treated with a single dose of the compound on day 0, 4, 7, 14, a) duration of the irritant effect expressed in [s], b) pressure of the irritant expressed in grams [g]. Significance level p*<0.01, p**<0.001, p ****<0.0001.

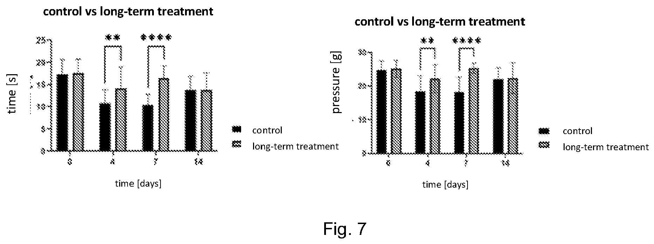

Figure 7 shows analgesic effect of the compound following central i.t. administration in the chronic pain model of ligation of the sciatic nerve and measurement of the level of mechanical hypersensitivity tested with the von Frey test in control rats in relation to the group of rats treated with daily dose of the compound for 14 days, a) duration of the irritating stimulus expressed in seconds [s] , b) pressure of the irritant expressed in grams [g]. Significance level p**<0.001, p****<0.0001.

### Example 1. Preparation of the peptide according to the invention.

The H₂N-Tyr-D-Ala-Gly-Phe-Gly-Tyr-Pro-Ser-D-Ala-Phe-OH peptide was synthesized manually on a polymer support (Wang's resin) using the Fmoc (9-fluorenylmethoxycarbonyl) protecting group strategy. The amino acid coupling cycle was performed using 2-(1H-benzotriazol-1-yl)-1,1,3,3-tetramethyluronium tetrafluoroborate (TBTU), N-hydroxybenzotriazole (HOBt), and N,N-diisopropylamine (DIPEA). Reactions were carried out in dimethylformamide (DMF). The effects of attaching next amino acids and cleaving the Fmoc group were checked by the Kaiser test or the chloranil test (Pro). The Fmoc protecting group was removed using a 20% solution of piperidine in DMF. After the synthesis on the polymer support was completed, the peptide was cleaved from the resin with trifluoroacetic acid (TFA). The peptide was precipitated with cold diethyl ether, centrifuged, dried and then purified using reverse phase high performance liquid chromatography (RP-HPLC). Separation was performed on a Jupiter^{®} 10 µm Proteo 90 Å, AXIA^{™}, C12 column (250 x 21.2 mm) using mobile phases A: 0.1% TFA in water and B: 0.1% TFA in acetonitrile, at 10 ml/min flow in a gradient of 30 - 50% of phase B, in 20 min. Product containing fractions were lyophilized, and characterized by analytical HPLC coupled to a mass spectrometer (LC-MS). The analytical HPLC system used a Jupiter 4 µm Proteo 90 Å, C12, (250 x 4.6 mm) column. A linear gradient of 3-14% of phase B in 12 min was applied (phase A: 0.05% formic acid in water, phase B: 0.05% formic acid in acetonitrile), flow rate 1.2 ml/min. Product retention time 11.94 minutes. Characterization by mass spectrometry (ESI) confirmed the molecular weight of the peptide: calculated [M+H]+ 1021.48, found 1021.85, calculated [M+2H]2+ 511.23, found 511.80.

### Example 2. Analgesic effect of the peptide according to the invention.

The effect of the peptide according to the invention in comparison to dermorphin was tested. Thus, dermorphin and the peptide according to the invention were dissolved in physiological saline and administered to rats subarachnoid space according to the method described by Yaksh & Rudy (Yaksh T.R., Rudy T.A., Physiol. Behav. 17: 1031-1036 (1976)), in two doses 0.04, and 0.4 nmol/kg. The analgesic effect was calculated as a percentage of the maximum possible analgesic effect, which influences the pain induced in rat by an external stimulus (% MPE). The doses were selected in such a way as to show the analgesic effect in comparison to a moderate and maximum effect of dermorphin. The studies demonstrated that at equivalent doses the peptide according to the invention is as potent as dermorphin. The results are shown in Fig. 1. At a lower dose, this effect is slightly higher in the first 30 minutes than for dermorphin, while at a higher dose it is equipotent for the first 30 minutes, and then the effect diminishes compared to dermorphin, although the value of MPE was maintained for 120 minutes after administration, at a high, 40 % level.

In the next step, the dose-dependent effect of the peptide according to the invention was tested in the dose range from 0.004 nmol/kg to 0.4 nmol/kg. An increase in the analgesic effect depending on the used dose, was observed. The threshold dose of 0.004 nmol/kg was active at a low but noticeable level. The 10-fold increase in the dose produced a marked spike in the analgesic effect. The doses of 0.04 nmol/kg and 0.08 nmol/kg significantly increased the analgesic effect and extended its duration, initially to 30 and then to 60 minutes after administration. The results are shown in Fig. 2. The highest doses of 0.08 nmol/kg and 0.4 nmol/kg achieved the maximum effects lasting up to 30 min, and in 60 and 120 min the activity decreased to the value of 60 - 70% MPE. However, these values are also exceptionally high, when comparing activity of the tested compound to that of morphine at the equipotential dose. A similar effect is produced by morphine at a dose approximately 100 times higher (32.0 nmol/kg) than that of the peptide according to the invention. The results are shown in Fig. 3.

The obtained results, presented in Figs. 1-3, prove that the peptide according to the invention is as potent as dermorphin, and definitely more potent than morphine.

### Example 3. Stability of the peptide according to the invention in human serum.

The stability of the peptide according to invention in comparison to dermorphin was tested. For this purpose, the peptide stock solution at a concentration of 34 nmol/µl, containing additionally two internal standards, i.e. H-Trp-OH and Z-Val-OH was prepared. A 1:1 (v/v) mixture of water and acetonitrile was used as a solvent. The stock solution was then diluted 25 times with water, thereby giving a working solution suitable for the metabolic stability study. Next, the working solution of the peptide was mixed 1:1 (v/v) with human blood serum, and incubated at 37°C while stirring (300 rpm). At selected time intervals, i.e. after 0 min, 24 h, 48 h, 72 h, and 96 h from the start of incubation, samples of the degradation mixture (100 µl) were taken, and quenched with 96% ethyl alcohol (200 µl) to stop the degradation process. After that, the so obtained mixtures were centrifuged (15 min. at 4°C ,at 14,000 rpm), which allowed for separation of the supernatant from the sediment of proteins contained in the blood serum. From the obtained supernatant, 160 µL was taken and analyzed by RP-HPLC and RP-HPLC-MS. Analysis of the results (HPLC and HPLC-MS) allowed for plotting concentration of non-degraded peptide (substrate) in the degradation mixture as a function of time, in relation to the initial content (%, for t=0 min the concentration is 100%).

The obtained results of the experiment are shown in Fig. 4, the experiment was repeated twice for each measurement point. As a result of the study, it was found that the biological half-life time t_{1/2} of the peptide according to the invention in human serum is greater than 96 h. The known stability of pure dermorphin in human or rat serum is much shorter than that observed for the peptide according to the invention, and according to the available knowledge is approximately 170-190 min (Scalia S. et al. Peptides, 7: 247-251 (1986), Biondi L, et al. J. Peptide Sci. 9: 638-648 (2003).

### Example 4. Peptide analgesic effect in the chronic constriction injury (CCI) model.

In the next step of the study, the peptide was administered by the central (subarachnoid) route to rats with induced neuropathic pain (the model of neuropathic pain of loose ligation of the sciatic nerve according to Bennett [Bennett and Xie, 1988]) according to the method described by Yaksh & Rudy (Yaksh T.R., Rudy T.A., Physiol Behav. 17: 1031-1036 (1976). In the first step, the level of pain experienced by rats with chronic pain was determined in comparison to healthy rats. The results of the test are shown in Fig. 5. The behavioral test consisted of measuring the level of allodynia on the pelvic, "sick" limb, where the time or strength of the irritating stimulus acting on the bottom of the foot were tested.

In the main test of the analgesic effect of the tested peptide, a single dose of the peptide was administered by the central route at predetermined time intervals. To that end, the compound was dissolved in physiological saline solution and administered by a Hamilton syringe to the rats on days 0, 4, 7, 14 of the experiment. Earlier studies let to establish that in our case, the effective dose of peptide for rats is 2.0 nmol/kg. Results of the test clearly proved that with this method of administration, the compound is highly analgesic on days 4, 7 and 14 of pain induction, i.e. during the period of intense neuropathic pain sensation, Fig. 6. The level of perceived pain in the treated rats was similar to the level of pain on day 0, that is on the day the test was started, and when neuropathic pain had not yet developed, Fig. 6.

In the treatment of chronic pain, pharmacotherapy is based on repeated administration of the active substance, which is often associated with the occurrence of the tolerance and undesired side effects. Therefore, in the next step of the study, the analgesic compound was administered to the rats daily for a period of 14 days, Fig. 7.

The results clearly demonstrated that the tested rats did not develop tolerance after 14 days of administration of the compound. On days 4 and 7, the compound was highly analgesic, in comparison to the untreated group. On the fourteenth day, the compound did not induce its analgesic effect, in comparison to the control group, but at the same time it did not induce hyperalgesia, which is commonly associated with long-term administration of opioids, e.g. morphine. In addition, the effects that commonly appear during chronic opioid administration in animals, and are indicative of the development of opioid tolerance, were not observed in rats administered daily with the compound. These mentioned above undesired side effects include weight loss, hyperalgesia to touch, vocalization, and hair shedding. In this case, the rats were in good general physical condition and did not show the above-mentioned symptoms.

### Summary and Conclusions

Based on the experiments described above, the following conclusions can be drawn about the properties of the peptide according to the invention:
- the compound is stable in the blood plasma,
- the compound shows high analgesic activity in the acute pain model, after central i.t. administration (subarachnoid),
- the compound shows high analgesic activity in a model of neuropathic pain, after a single, central (subarachnoid) administration,
- the compound shows high analgesic activity in a neuropathic pain model, after the central i.t. (subarachnoid) administration, and does not induce a tolerance when administered daily for 14 days,
- when the compound was used in the 14-day test in rats, no behavioral symptoms suggesting an adverse effect of being on the substance during this period were observed.

## Claims

1. The peptide with the sequence:
H₂N-Tyr-D-Ala-Gly-Phe-Gly-Tyr-Pro-Ser-D-Ala-Phe-OH.

2. The peptide according to claim 1 for use in pharmacy.

3. The peptide according to claim 1 for use as analgesic.

4. The peptide for use according to claim 3, **characterized in that** it is intended for a long-term analgesic therapy.

5. The peptide for use according to claim 3, **characterized in that** it is intended for administration to the patient at 1 to 2 days intervals.

6. The peptide for use according to claim 3, **characterized in that** it is administered parenterally, preferably intrathecally.

7. The peptide for use according to claim 3, **characterized in that** it is intended to be administered at a dose at least 100 times lower than the analgesic, effective dose of morphine **in that** patient.

8. The peptide for use according to claim 3, **characterized in that** it is intended to be administered once daily at a dose not greater than 0.4 nmol/kg.

## Patentansprüche

1. Peptid mit der Sequenz:
H₂N-Tyr-D-Ala-Gly-Phe-Gly-Tyr-Pro-Ser-D-Ala-Phe-OH.

2. Peptid nach Anspruch 1, zur Verwendung in der Pharmazie.

3. Peptid nach Anspruch 1, zur Verwendung als Analgetikum.

4. Peptid zur Verwendung nach Anspruch 3, **dadurch gekennzeichnet, dass** es für eine analgetische Langzeittherapie bestimmt ist.

5. Peptid zur Verwendung nach Anspruch 3, **dadurch gekennzeichnet, dass** es für die Verabreichung an den Patienten in Intervallen von 1 bis 2 Tagen bestimmt ist.

6. Peptid zur Verwendung nach Anspruch 3, **dadurch gekennzeichnet, dass** es parenteral, bevorzugt intrathekal, verabreicht wird.

7. Peptid zur Verwendung nach Anspruch 3, **dadurch gekennzeichnet, dass** es dazu bestimmt ist, in einer Dosis verabreicht zu werden, die mindestens 100-mal niedriger ist als die analgetisch wirksame Dosis von Morphin bei diesem Patienten.

8. Peptid zur Verwendung nach Anspruch 3, **dadurch gekennzeichnet, dass** es dazu bestimmt ist, einmal täglich in einer Dosis von nicht mehr als 0,4 nmol/kg verabreicht zu werden.

## Revendications

1. Peptide ayant la séquence :
H₂N-Tyr-D-Ala-Gly-Phe-Gly-Tyr-Pro-Ser-D-Ala-Phe-OH.

2. Peptide selon la revendication 1 pour une utilisation en pharmacie.

3. Peptide selon la revendication 1 pour une utilisation comme analgésique.

4. Peptide pour une utilisation selon la revendication 3, **caractérisé en ce qu'**il est destiné à une thérapie analgésique à long terme.

5. Peptide pour une utilisation selon la revendication 3, **caractérisé en ce qu'**il est destiné à une administration au patient à des intervalles allant de 1 à 2 jours.

6. Peptide pour une utilisation selon la revendication 3, **caractérisé en ce qu'**il est administré par voie parentérale, de préférence par voie intrathécale.

7. Peptide pour une utilisation selon la revendication 3, **caractérisé en ce qu'**il est destiné à être administré à une dose au moins 100 fois inférieure à la dose analgésique efficace de morphine chez ce patient.

8. Peptide pour une utilisation selon la revendication 3, **caractérisé en ce qu'**il est destiné à être administré une fois par jour à une dose ne dépassant pas 0,4 nmol/kg.
